# EUROPEAN PATENT SPECIFICATION

(11) **EP 1 189 519 B1**
(45) Date of publication and mention of the grant of the patent: **08.04.2009**
(21) Application number: 01901148.5
(22) Date of filing: 10.01.2001
(51) Int. Cl.: A23K 1/18, A61Q 11/00

(54) **DENTAL DIET FOR REDUCING TARTAR**
ZAHNÄRZTLICHE DIÄT ZUR VERMINDERUNG DES ZAHNSTEINS
REGIME DENTAIRE POUR LA REDUCTION DU TARTRE

(30) Priority: 14.01.2000 US 483328
(43) Date of publication of application: 27.03.2002
(73) Proprietor: Société des Produits Nestlé S.A., 1800 Vevey (CH)
(72) Inventor: CUPP, Carolyn, Jean, Liberty, MO 64068 (US); GERHEART, Lynn, Ann, Smithville, MO 64089 (US); SCHNELL, Scott, St Joseph, MO 64506 (US); SMITHEY, Sheri, Lynn, St. Joseph, MO 64501 (US); ANDERSON, Donna, Elizabeth, Weatherby Lake, MO 64152 (US); DIXON, Dan, St. Joseph, MO 64505 (US)
(74) Representative: Lock, Graham James
(86) International application number: PCT/EP2001/000307
(87) International publication number: WO 2001/050882

(56) References cited:
- EP-A- 0 575 021
- EP-A- 0 749 695
- EP-A- 0 909 536
- US-A- 4 800 099

## Description

### BACKGROUND OF THE INVENTION

The present invention relates generally to pet foods. More specifically, the present invention relates to pet foods that when chewed by pets, cause a mechanical, abrasive cleaning of the pets' teeth.

Many pets suffer from dental health problems. It is estimated that over 70% of dogs have some degree of gingival or periodontal disease. Plaque formation on the surface of the pets' teeth is a primary factor in the development of such problems. Dental plaque is a combination of bacteria, bacterial by-products which form a glycoprotein matrix, and salivary precipitates which bind to the tooth surface.

Contributing to the problem is the formation of tartar or dental calculus (mineralized plaque). Dental calculus forms on the tooth surface at or above the gum line and serves as a substrate for the additional accumulation of plaque. Apart from causing gum irritation and potentially, periodontal disease if left untreated, this calculus has an unsightly appearance.

Pets are more susceptible to the formation of plaque and calculus than animals in the wild. This is due to the nature of the foods that pets eat. In the wild, many animals eat foods that mechanically abrade plaque and calculus from the teeth. In contrast, pets are usually fed commercially available pet foods that although they may provide better nutritional value, do not in general subject the teeth to abrasive forces sufficient to clean the teeth. This is even true of dried kibbles that are able to abrade the teeth only to a very limited extent. In part, this is due to the fact that dried kibbles usually crumble when chewed by the pet.

Maintenance of optimal dental health depends on regular professional cleaning of all these accumulations both above and below the gum line, as well as adequate home care. Mechanical cleansing of the tooth surfaces with tooth brushing is an effective means of controlling the buildup of plaque and calculus in both humans and pets. However, most pet owners are reluctant or unable to provide the routine brushing necessary to maintain good oral health in their pets and would likely welcome an alternative provided by dietary means.

There have been various attempts to provide products for improving pet dental health. One such attempt centers around the use of chews made from rawhide or rawhide substitutes. By allowing the pet to gnaw or chew on such rawhide products, the pet abrades calculus and plaque from its teeth. It is also possible, as described in European patent application 0272968, to incorporate various oral care agents into such chews. These products, however, have the drawback that they are typically expensive. Moreover, they are usually only effective with dogs that are prone to chew and gnaw.

Another approach has been to incorporate oral care agents into certain pet foods. For example, U.S. Patent No. 5,000,940 discloses baked dog biscuits which contain a tetrasodium pyrophosphate salt. The patent states that the salt causes a reduction in calculus accumulation. Another example of such an approach is, European patent application 0205354 which discloses baked dog biscuits which contain vegetable fibers to abrade the teeth of the dog when chewed. A drawback with both of these products, however, is that the biscuits crumble upon being bitten. Hence, the dog cannot chew the product and little abrasion occurs. This thereby reduces the efficacy of the product.

A further approach is described in U.S. Patent No. 5,431,927 (EP 0 575 021). That patent describes a dried product which contains aligned fibers which, when chewed, fracture in long striations rather than crumbling. The patent states that this allows the product to remain in contact with the animals' teeth for a longer period of time hence enhancing the abrasive effect. However, a disadvantage of this product is that it must be produced using a specially coated die that allows laminar flow conditions within the die. The laminar flow condition is reported to cause the alignment of the fibers within the product leading to the fracturing of the product when bitten by the animal. The use of such dies necessitates a complicated procedure for manufacturing the product.

Lamellar pet food products are also described in EP 0 749 695. However the products disclosed therein do not demonstrate oral care properties. Canine biscuits having dental care properties are disclosed in US 4 800 099.

The biscuits contain discrete particles of meat or the like to enhance palatability.

EP 0 909 536 also discloses products having oral care properties. The biscuits disclosed therein have a density of 285 kg/m³ and are formed form Kibbles having an elliptical shape with a short diameter of 11 mm, a long diameter of 12 mm and a length of 9 mm.

There is therefore a need for an improved dental care pet food.

### SUMMARY OF THE INVENTION

The present invention provides a dry pet food that will reduce tartar when chewed by the pet. It has been surprisingly found that by reducing the density and/or increasing the size of the pet food product, that the resultant product will remove more plaque and tartar build-up than similar pet food products.

A first aspect of the invention provides a pet food composition comprising by dry weight 12 to 50% denatured protein including corn gluten meal, 2 to 15% insoluble fibre, 20 to 65% gelatinized carbohydrate and 0 to 5% of a humectant for use in a method for reducing calculus and plaque build up on a pet teeth, the method comprising allowing a pet to chew on the dried pet food characterised in that the pet food has a density of less than 330 kg/m³, a length of at least 15 mm, a width of at least 13.5 mm and a thickness of at least 12 mm.

In one embodiment of the present invention, a dried pet food is provided comprising at least 25% by weight of a kibble including a matrix having a protein source, carbohydrate source, insoluble fiber, and a density of less than 330 kg/m³ (20.5 lbs/ft³) as described above.

In another embodiment of the invention the insoluble fiber may be a cellulose fiber.

Pet food compositions are also provided. A second aspect of the invention provides a pet food composition comprising 12 to 50% denatured protein including corn gluten meal, 2 to 15% by weight insoluble fibre, 20 to 65% by weight gelatinised carbohydrate and 0 to 5% by weight of a humectant, the pet food having a density of less than 330 kg/m³ and characterised in that the pet food is provided as a Kibble having a length of at least 15 mm, a width of at least 13.5 mm and a thickness of at least 12 mm.

An advantage of the present invention is to provide an improved pet food for reducing tartar on pets' teeth.

Another advantage of the present invention is to provide an improved dental care pet food for dogs.

A still further advantage of the present invention is to provide an improved dental care product that can be mixed with regular pet food and still achieve dental health care benefits for the pet.

Moreover, an advantage of the present invention is to provide a dried pet food that does not require the use of a humectant.

Furthermore, an advantage of the present invention is to provide an improved method for making dried pet food that provides dental benefits.

Further, an advantage of the present invention is to provide a cost effective method for improving the dental health of pets.

Additional features and advantages of the present invention are described in and will be apparent from the detailed description of the presently preferred embodiments set forth below.

### BRIEF DESCRIPTION OF DRAWINGS

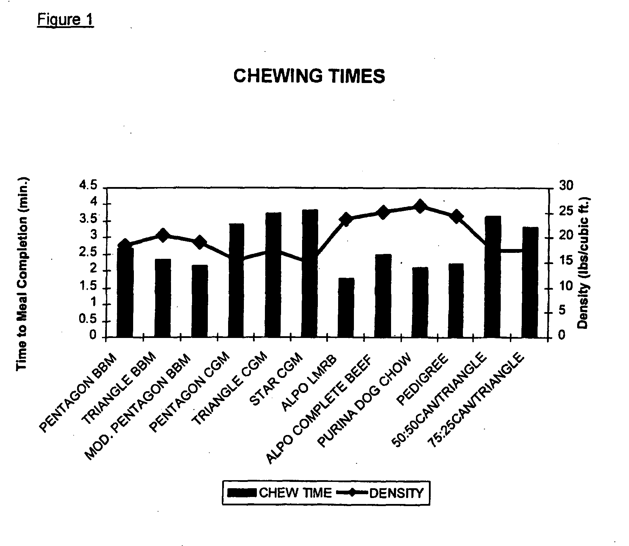

Figure 1 is a graph showing a comparison of chewing time of the inventive product with chewing times of currently available products.

### DETAILED DESCRIPTION OF THE PRESENTLY PREFERRED EMBODIMENTS

The present invention relates generally to dried pet food for cleaning pets' teeth. It has been surprisingly found that by reducing the density of dried pet food and/or providing a larger product, that an improved tartar removing product can be provided for pets. This is true even if the product does not include a texturizing agent (humectant).

The pet food has a density of approximately 15.6 lb/ft³ (250 kg/m³) to about 20.5 lb/ft³ (330 kg/m³) preferably, approximately 16.8 lb/ft³ (270 kg/m³) to 20 lb/ft³ (320 kg/m³). The pet food is dimensional such that it has a length of at least 15 mm, a width of at least 13.5 mm, and a thickness of at least 12 mm. In an embodiment, the pet food has a length of approximately 20 mm, a width of at least 15 mm, and a thickness of at least 18 mm. The pet food may alternatively be provided in kibble shapes such as triangles, pentagons and stars. An example of a triangle kibble has the following dimensions : thickness 16mm, base 28mm and sides 32mm. An example of a pentagon has a diameter of 22.8mm and a thickness of 14.2mm.

Preferably, the pet food has a texture such that a probe, having a contact area of about 1 mm² and operated at a speed of about 5 mm/s, penetrates into the matrix for a distance of at least about 30% of the thickness of the matrix prior to breaking the matrix, more preferably at least about 40%. For example, for a dog food, the probe may penetrate a distance of approximately 6.0 mm, preferably at least 6.5 mm, prior to breaking the matrix.

The carbohydrate source is conveniently a grain such as corn, rice, wheat, beets, barley, oats, or soy, and/or mixtures of these grains. The grain is typically provided in the form of a flour or a meal. Pure or substantially pure starches may also be used if desired. The exact carbohydrate source or sources used in the present invention is not critical to the invention. Generally, the carbohydrate source is selected on the basis of cost and palatability. It should be noted that the carbohydrate source may or may not contain protein.

A variety of protein sources can be used for the protein component. The protein source may be a vegetable protein source, an animal protein source, or a mixture of these protein sources. Suitable vegetable protein sources include gluten, wheat protein, soy protein, rice protein, corn protein, and the like. These proteins can be provided in the form of flours, concentrates, and isolates, as desired. Suitable animal protein sources include muscular or skeletal meat of mammals, poultry, and fish; meals such as meat meal, bone meal, fish meal, and poultry meal; by-products such as hearts, liver, kidneys, tongue, and the like; and milk proteins. The dried pet food contains approximately 12% to about 50% by weight proteins, and in a preferred embodiment, more than about 15% to 40% by weight protein. The dried food may contain a mixture of protein sources, for example, corn gluten meal and beef and bone meal. It is found to be advantageous to include corn gluten meal at about 15 - 20% together with beef and bone meal at 3 - 10% to obtain the desired density of the final product. The protein sources may also include soy bean meal. A preferred range for soy bean meal is from only 5% to 15%, more preferably 6 - 8% of the total composition of the food mix formulation.

The insoluble fiber may be any suitable fiber. By way of example, suitable fibers include soy fiber, rice hull fiber, pea hull fiber, oat hull fiber, barley hull fiber, sugarbeet fiber, wheat bran fiber, and pure cellulose. One such cellulose fiber is Solka-Floc®. Generally, the fiber is selected on the basis of cost and palatability considerations. However, as noted above, the product should have a density less than 330 kg/m³. Accordingly, a fiber must be selected which results in a lower density product. Accordingly, a cellulose fiber may be used in a preferred embodiment. If used, preferably, the dried pet food contains approximately 2% to about 15% by weight of insoluble fiber.

As noted above, a texturizing agent is not necessary. However, if a texturizing agent is desired, a humectant may be provided. The humectant may be any suitable humectant, for example, glycerin, propolyne glycol, butylene glycol, polyhydric glycols such as glycerol and sorbitol, hydrogenated starch, hydrolysates and the like. If used, the dried pet food can contain up to 5% by weight humectant.

If desired, abrasive agents may also be included. Suitable abrasive agents include ground oyster shells, titanium dioxide, and the like. Similarly dental care agents may also be used if desired; for example, pyrophosphate salts such as tetrasodium pyrophosphate.

Various other ingredients, for example, salt, spices, seasonings, vitamins, minerals, flavoring agents, lipids, and the like may also be incorporated into the dried pet product as desired. If added, the lipids may be any suitable animal fat; for example tallow, or may be a vegetable fat.

The dried pet food may be manufactured in many different ways as desired. However extrusion gelatinization is found to be particularly suitable.

In a specific example of a suitable extrusion gelatinization process, a dry feed mixture is prepared from a protein source, a carbohydrate source, insoluble fiber, vitamins, and minerals. The dry feed mixture is then fed into a preconditioner.

In the preconditioner, water or steam, or both, is mixed into the dry feed mixture. Further, liquid flavor components, such as flavor digests or tallow, may be mixed into the dry feed mix in the preconditioner. Sufficient water, steam, or liquid flavor components is mixed into the feed mixture to raise the moisture content of the dry feed mixture to approximately 10% to about 30% by weight. If desired, the temperature of the dry feed mixture may be raised in the preconditioner to approximately 60°C to about 95°C. A suitable preconditioner is described in U.S. Patent No. 4,752,139.

The moistened feed leaving the preconditioner is then fed into an extruder. If the product will include a humectant, the humectant is conveniently added to the moistened feed in the extruder. The extruder may be any suitable single or twin screw, cooking-extruder. Suitable extruders may be obtained from Wenger Manufacturing Inc., Clextral SA, Bühler AG, and the like. During passage through the extruder, the moistened feed passes through a cooking zone, in which it is subjected to mechanical shear and is heated to a maximum temperature of up to about 130°C, and a forming zone. The gauge pressure in the forming zone is approximately 600 kPa to about 10 Mpa as desired. If desired, water or steam, or both, may be introduced into the cooking zone. Further, during passage through the extruder, the starch ingredients of the moistened feed are gelatinized to provide a gelatinized matrix of starch, protein, insoluble fiber, and humectant.

The gelatinized matrix leaving the extruder is forced through a die. Any suitable die may be used. However, the orifice of the die is preferably chosen such that the distance from the center of the orifice to any inner surface is at least about 5 mm. This ensures that the extrudate has a diameter of at least about 18 mm; more preferably at least about 20 mm. Further, the orifice is preferably shaped to provide a product kibble having at least one corner. Suitable examples of shapes are found to include triangles, pentagons and stars.

Upon leaving the die, the extrudate is cut into pieces using blades. The blades are preferably arranged such that the pieces have a length of at least about 12 mm; for example about 14 mm. The individual pieces may then be processed as desired. For example, they may be partially or fully dried and coated with further flavoring agents. After cooling, the pieces may be packed into suitable packages.

After drying, the pieces preferably have a moisture content of less than about 10% by weight; for example about 3% to about 7% by weight when leaving the drier. The pieces preferably have a water activity of less than about 0.7; more preferably less than about 0.6.

It has been found that the dry dog food kibbles of the invention require increased chewing time by the pet, assisting in the increasing of the dental benefits to be derived from the food. It is also noted that the length of chewing bouts increases, providing exercise for jaw muscles. This is believed to contribute to improved oral health.

By way of example, and not limitation, examples of the present invention will now be given.

### Example No. 1A

A dry mix is prepared from about 62% by weight of whole corn, about 16% by weight of beef and bone meal, about 14% soybean meal, about 4% by weight of fish and poultry meals, and about 3% by weight of cellulose and various vitamins and minerals. The dry mix is fed into a preconditioner along with a flavor digest. The preconditioned mixture is then fed into an extruder with or without about 2% by weight of glycerin. The preconditioner is operated at about 87°C. Steam is injected into the preconditioner at about 9% DMR and water at about 4% DMR.

The moistened feed leaving the preconditioner is then fed into a model 165 extruder obtained from Wenger and gelatinized. The extruder has six zones and the temperatures in the six zones are about 87°C, about 87°C, about 102°C, about 101°C, about 102°C, and about 127°C. The pressure upon leaving the extruder is about 3.4 MPa gauge.

The gelatinized mixture is forced through the orifice of a die. The orifice is in the shape of a circle in cross section with a diameter of 10 mm. The extrudate leaving the die is cut into pieces of 14 mm length. The pieces are then coated with flavoring agents and dried in the normal manner.

The pieces have a density of about 328 kg/m³ and a moisture content of about 8.5% by weight.

### Example No. 1B

A second dry mix is prepared and extrusion cooked, cut and flavor coated as described above in Example 1A to produce a second pet food, except that instead of having the beef and bone meal of Example 1A, the dry mix contains 16% by weight of corn gluten meal. The product had a density of about 280 kg/m³.

### Example No. 2

An amount of 50 dried pieces obtained using the process of Example 1A are subjected to texture analysis using a TA-XT2 Texture Analyzer obtained from Stable Micro Systems, Inc. The Texture Analyzer is fitted with a rod-like probe which has a length of about 52 mm. The probe is made up of two sections; a first section and a second section. The first section has a length of about 21 mm and a constant diameter of about 9.5 mm. The second section tapers down to a point having a contact area of about 1 mm². The Texture Analyzer is operated at a speed of 5 mm/s and a contact force of 5 g.

Each piece is placed on a base under the probe. The probe is moved downwardly and into the piece. The distance of penetration of the probe into the piece, the compression force and the time are recorded at a rate of 200 recordings per second. Breakage of the piece is determined upon a sharp fall off of the compression force. The distance of penetration, the compression force and the time are recorded at the moment of breakage. The values obtained for all pieces are then averaged.

For comparison, the process is repeated for each group of 50 pieces of products used in the dental trial (Example 3). The results are as follows:

| Product | Distance to breakage /mm |
|---|---|
| Example 1A | 6.56 |
| Example 1B | 9.50 |
| Purina Dog Chow | 3.30 |

The results indicate that the pieces of Examples No. 1A and 1B are significantly better than standard dry dog foods.

Because the products of Examples 1A and 1B are resistant to breakage, the animal needs to bite deeper into each piece of the product before it breaks. Therefore the animal's teeth are subjected to improved mechanical cleaning.

### Example No. 3

A group of 36 healthy adult dogs are used in this trial. Each animal is given a complete veterinary physical examination. Oral exams are also conducted to select only dogs without obvious dental/oral problems. The dogs are divided into three groups of 12 dogs each with an even distribution of age, sex, and susceptibility to calculus formation in each group. During the trial, the dogs have ad libitum access to water and food and are fed once daily. The food consumption of each dog is monitored daily. The weight of each dog is recorded at the start of the trial and again each week.

The trial is initiated by performing complete dental prophylaxis on all dogs to carefully remove (by ultrasonic teeth cleaning) all supra- and sub-gingival deposits of plaque and calculus. Also, the dogs' teeth are thoroughly polished. Each group of dogs is then randomly allocated a different food product and fed that product for the duration of the trial. Group 1 is fed the pieces of Example No. 1A, Group 2 is fed the second dental prototype of Example No. 1B, and Group 3 is fed Purina Dog Chow.

After 7 days of feeding, dogs are sedated and a few drops of a 3% erythrosin plaque-disclosing solution are applied to the teeth of each dog and then thoroughly rinsed off with tap water. Plaque evaluation is then carried out on gingival and occlusal halves of the upper third incisors, upper and lower canines, upper 3^{rd} and 4^{th} premolars, upper first molars, lower 3^{rd} and 4^{th} premolars, and lower first molars. An assessment of the buccal tooth surface that is covered with plaque is made according to the following scale:
0 = No observable plaque;
I = Plaque covering less than 25% of the tooth surface;
2 = Plaque covering between 25% and 50% of the tooth surface;
3 = Plaque covering between 50% and 75% of the tooth surface;
4 = Plaque covering greater than 75% of the tooth surface.

Plaque thickness is assessed as follows:
1 = Light or thin, a light pink color;
2 = Medium, a moderate or medium shade of red;
3 = Heavy or thick, a dark bright shade of red.

A score is then obtained by multiplying the coverage score by the thickness score for each half of the 14 teeth to give a score ranging from 0 to 12. The score for each half of a tooth are added to provide a whole tooth score. The whole tooth scores are then averaged.

On the 21^{st} day of the trial, evaluation of calculus is similarly performed for each animal on the proximal, mesial, and distal thirds of the 18 teeth previously examined. No disclosing solution is used to score calculus. The scores for each third of a tooth are added to provide a whole tooth score and all whole tooth scores are averaged. Concurrent with calculus scoring, a gingival index is performed in which one score is recorded per tooth based on the most severe portion of the tooth's gingival margin. Gingival index is assessed as follows:
0 = No inflammation or swelling;
1 = Mild inflammation, slight redness or swelling, no bleeding on gentle probing;
2 = Moderate inflammation, redness and swelling, bleeding on gentle probing;
3 = Severe inflammation, bright red and swollen gingiva, spontaneous bleeding on probing.

Scores for all animals in each group are averaged and the results are as follows:

| **Product** | **Plaque Score** | **Calculus Score** | **Gingival Index** |
|---|---|---|---|
| | **Day 7** | **Day 21** | **Day 21** |
| Example 1A Dental | 6.45 | 3.87 | 0.24 |
| Example 1B Dental | 6.93 | 4.06 | 0.24 |
| Purina Dog Chow | 7.79 | 5.33 | 0.46 |

The results indicate that the products of Example No. 1 show significantly improved cleaning of the dogs' teeth over a commercially available dry dog food. These results correlate with those of Example No. 2.

### Example No. 4

A group of 36 healthy adult dogs are used in this trial. Each animal is given a complete veterinary physical examination. Oral exams are also conducted to select only dogs without obvious dental/oral problems. The dogs are divided into three groups of 12 dogs each with an even distribution of age, sex, and susceptibility to calculus formation in each group. During the trial, the dogs have ad libitum access to water and food and are fed once daily. The food consumption of each dog is monitored daily. The weight of each dog is recorded at the start of the trial and again each week.

The trial is initiated by performing complete dental prophylaxis on all dogs to carefully remove (by ultrasonic teeth cleaning) all supra- and sub-gingival deposits of plaque and calculus. Also, the dogs' teeth are thoroughly polished. Each group of dogs is then randomly allocated a different food product and fed that product for the duration of the trial. Group 1 is fed the pieces of Example No. 1B, in a 50:50 blend with standard-sized Alpo Complete pieces. Group 2 is fed the pieces of Example No. 1B, in a 25:75 blend with standard-sized Alpo Complete pieces (25% dental pieces). Group 3 is fed standard Alpo Complete dry dog food.

Plaque, calculus, and gingival scores are performed as in Example No. 3. Scores for all animals in each group are averaged and the results are as follows:

| **Product** | **Plaque Score** | **Calculus Score** | **Gingival Index** |
|---|---|---|---|
| | **Day 7** | **Day 21** | **Day 21** |
| 50% Dental | 7.69 | 3.29 | 0.45 |
| 25% Dental | 7.39 | 2.97 | 0.46 |
| Alpo Complete | 8.56 | 5.36 | 0.56 |

The results indicate that the two test products show significantly improved cleaning of the dogs' teeth over standard dry dog food.

### Example No. 5

Fifty dried pieces obtained using the inventive products of Example 4 were subjected to texture analysis as described in Example 2.

For comparison, the process is performed for each group of 50 pieces of dry products used in the dental trial (Example 4). The results are as follows:

| Product | Distance to Breakage/mm |
|---|---|
| ALPO Complete (nugget) | 2.1 |
| ALPO Complete (bone) | 1.2 |
| Example 4 (large pieces) | 6.7 |
| (density of 19.9 lbs/ft³) | |
| Example 4 (small pieces) | 2.3 |
| (density of 20.2 lbs/ft³) | |

The results indicate that the large pieces of Example 4 are significantly better than standard dried pet foods.

Because the products of Examples 1 and 4 are resistant to breakage, the animal needs to bite deeper into each piece of the product before it breaks. Therefore the animal's teeth are subjected to improved mechanical cleaning.

### Example No. 6

Eleven dogs (Labradors and beagles) are selected for a chewing time trial. Each day, a predetermined quantity of food is given to each dog, according to its individual metabolic/activity needs. Each day the food is different and the meal is individually timed to determine the time to completion by a particular participating dog. The different food meals and chewing times are shown in the accompanying graph, figure 1.

The formulations for the test diets are the following:
a. High beef and bone meal (BBM) formula, comprising 16% w/w beef and bone meal, 61.8% w/w corn, and being free of corn gluten meal, provided as kibbles having a pentagonal shape;
b. High beef and bone meal (BBM) formula, comprising 16% w/w beef and bone meal, 61.8% w/w corn, and being free of corn gluten meal, provided as kibbles having a triangular shape;
c. High beef and bone meal (BBM) formula, comprising 16% w/w beef and bone meal, 61.8% w/w corn, and being free of corn gluten meal, provided as kibbles having a modified (rounded corner) pentagonal shape;
d. High corn gluten meal (CGM) formula, comprising 16% w/w corn gluten meal, 61.8% w/w corn, and being free of beef and bone meal, provided as kibbles having a triangular shape;
e. High corn gluten meal (CGM) formula, comprising 16% w/w corn gluten meal, 61.8% w/w corn, and being free of beef and bone meal, provided as kibbles having a star shape;
f. High corn gluten meal (CGM) formula, comprising 16% w/w corn gluten meal, 61.8% w/w corn, and being free of beef and bone meal, provided as kibbles having a star shape;
g. Purina Dog Chow (available from the Ralston Purina company, USA);
h. Pedigree dog food;
i. The CGM formula above in kibbles having a triangle shape, but being mixed with standard ALPO puppy chunk and gravy product at ratio of 50% dry: 50% canned;
j. CGM formula triangle shape mixed with standard ALPO puppy chunk and gravy product at ratio of 75% canned:25% dry; and The same 11 dogs ate all products on consecutive days.

It was noted that the lower density food products had the longest chewing times, that standard dry products had the fastest chewing times, and that mixing the large triangle piece with canned food also resulted in extended chewing. See figure 1.

It should be understood that various changes and modifications to the presently preferred embodiments described herein will be apparent to those skilled in the art. Such changes and modifications can be made without departing from the spirit and scope of the present invention and without diminishing its attendant advantages. It is therefore intended that such changes and modifications be covered by the appended claims.

## Claims

1. A dried pet food composition comprising by dry weight 12 to 50% denatured protein including corn gluten meal, 2 to 15% insoluble fibre, 20 to 65% gelatinized carbohydrate and 0 to 5% of a humectant for use in a method for reducing calculus and plaque build-up on a pet's teeth, the method comprising allowing a pet to chew on the dried pet food the pet food having a density of less than 330 Kgm⁻³, a length of at least 15 mm, a width of at least 13.5 mm and a thickness of at least 12 mm.

2. A pet food according to claim 1, wherein the denatured protein comprises by weight of the food composition 15 to 20% corn gluten meal and 3 to 10% beef and bone meal.

3. A pet food according to claim 1 comprising about 16% by weight corn gluten meal, about 14% by weight soybean meal, about 4% by weight fish and poultry meals, about 8% by weight cellulose and various vitamins and minerals, 62% by weight whole corn and optionally 2% by weight of glycerine, and having a density of 280 kg/m³.

4. A dried pet food according to claim 1 to 3, wherein the pet food has a texture such that a probe having a contact area of 1mm² and operated at a speed of 5mm/s penetrates into the matrix for a distance of at least 30% of the thickness of the matrix prior to breaking of the matrix.

5. A pet food according to any one of the preceding claims, wherein the dried pet food has a water activity of less than 0.7.

6. A pet food according to any one of the preceding claims, wherein the dried pet food is mixed with a wet pet food.

7. A pet food according to claim 6, wherein the dried and wet pet food are mixed in a ratio of dry pet food to wet pet food of 50:50.

8. A pet food according to any one of claims 1 to 7 wherein the pet food composition is provided in kibble shapes selected from triangles, pentagons and stars.

9. A dried pet food composition comprising 12 to 50% denatured protein including corn gluten meal, 2 to 15% insoluble fibre, 20 to 65% gelatinized carbohydrate and 0 to 5% of a humectant, the pet food having a density of less than 330 Kgm⁻³ and **characterised in that** the pet food is provided in a Kibble having a length of at least 15 mm, a width of a least 13.5 mm and a thickness of at least 12 mm.

10. A dried pet food according to claim 9, wherein the denatured protein comprises by weight of the food composition 15 to 20% corn gluten meal and 3 to 10% beef and bone meal.

11. A dried pet food according to claim 9 or claim 10, wherein the denatured protein comprises by weight of the food composition 5 to 15% soy bean meal.

12. A dried pet food composition according to claim 9, comprising about 16% by weight corn gluten meal, about 14% by weight soybean meal, about 4% by weight of fish and poultry meals, about 3% by weight of cellulose and various vitamins and minerals, 62% by weight of whole corn and optionally about 2% by weight of glycerine, and having a density of 280 kg/m³.

## Patentansprüche

1. Getrocknete Haustiernahrung, umfassend nach Trockengewicht 12 bis 50 % denaturiertes Protein einschließlich Getreideglutenmehl, 2 bis 15 % nicht lösliche Faser, 20 bis 65% gelatiniertes Kohlenhydrat und 0 bis 5 % Befeuchtungsmittel zur Verwendung in einem Verfahren zur Verminderung einer Zahnstein- und Plaquebildung an Haustierzähnen, das Verfahren umfasst, Ermöglichen einem Haustier auf der getrockneten Haustiernahrung zu kauen, wobei die Haustiernahrung eine Dichte von weniger als 330 Kgm⁻³, eine Länge von mindestens 15 mm, eine Weite von mindestens 13,5 mm und eine Dicke von mindestens 12 mm aufweist.

2. Haustiernahrung nach Anspruch 1, worin das denaturierte Protein 15 bis 20 Gew.-% der Nahrungszusammensetzung Getreideglutenmehl und 3 bis 10 Gew.- % Rindfleisch- und Knochenmehl umfasst.

3. Haustiernahrung nach Anspruch 1, umfassend ungefähr 16 Gew.-% Getreideglutenmehl, ungefähr 14 Gew.-% Sojabohnenmehl, ungefähr 4 Gew.-% Fisch- und Geflügelmehl, und 3 Gew.-% Cellulose und verschiedene Vitamine und Mineralien, 62 Gew.-% Vollkorn und wahlweise 2 Gew.-% Glycerin, und weist eine Dichte von 280 Kg/m³ auf.

4. Getrocknete Haustiernahrung nach Anspruch 1 bis 3, worin die Haustiernahrung eine Textur aufweist, so dass eine Sonde mit einer Kontaktfläche von 1 mm² und mit einer betriebenen Geschwindigkeit von 5 mm/s für einen Abstand von mindestens 30 % der Dicke der Matrix vor einem Brechen der Matrix in die Matrix eindringt.

5. Haustiernahrung nach einem der vorstehenden Ansprüche, worin die getrocknete Haustiernahrung eine Wasseraktivität von weniger als 0,7 aufweist.

6. Haustiernahrung nach einem der vorstehenden Ansprüche, worin die getrocknete Haustiernahrung mit einer nassen Haustiernahrung gemischt wird.

7. Haustiernahrung nach Anspruch 6, worin die getrocknete und nasse Haustiernahrung in einem Verhältnis von getrockneter Haustiernahrung zu nasser Haustiernahrung von 50:50 gemischt werden.

8. Haustiernahrung nach einem der Ansprüche 1 bis 7, worin die Haustiemahrungszusammensetzung in Trockenfutterformen ausgewählt unter Dreiecken, Fünfecken und Sternen bereitgestellt werden.

9. Getrocknete Haustiernahrungszusammensetzung, umfassend 12 bis 50 % denaturiertes Protein einschließlich Getreideglutenmehl, 2 bis 15 % nicht lösliche Faser, 20 bis 65 % gelatiniertes Kohlenhydrat und 0 bis 5 % eines Befeuchtungsmittels, wobei die Haustiernahrung eine Dichte von weniger als 330 Kgm⁻³ aufweist und **dadurch gekennzeichnet ist, dass** die Haustiernahrung in einem Trockenfutter mit einer Länge von mindestens 15 mm, einer Weite von mindestens 13,5 mm und einer Dicke von mindestens 12 mm bereitgestellt wird.

10. Getrocknete Haustiernahrung nach Anspruch 9, worin das denaturierte Protein 15 bis 20 Gew.-% der Nahrungszusammensetzung Getreideglutenmehl und 3 bis 10 Gew.-% Rindfleisch- und Knochenmehl umfasst.

11. Getrocknete Haustiernahrung nach Anspruch 9 oder Anspruch 10, worin das denaturierte Protein 5 bis 15 Gew.-% der Nahrungszusammensetzung Sojabohnenmehl umfasst.

12. Getrocknete Haustiernahrungszusammensetzung nach Anspruch 9, umfassend ungefähr 16 Gew.-% Getreideglutenmehl, ungefähr 14 Gew.-% Sojabohnenmehl, ungefähr 4 Gew.-% Fisch- und Geflügelmehl, und ungefähr 3 Gew.-% Cellulose und verschiedene Vitamine und Mineralien, 62 Gew.-% Vollkorn und wahlweise ungefähr 2 Gew.-% Glycerin, und weist eine Dichte von 280 Kg/m³ auf.

## Revendications

1. Composition alimentaire déshydratée pour animaux de compagnie, comprenant, en poids sec, 12 à 50 % de protéine dénaturée comprenant de la farine de gluten de maïs, 2 à 15 % de fibre insoluble, 20 à 65 % de glucide gélatinisé et 0 à 5 % d'un agent humidifiant, destinée à être utilisée dans une méthode pour réduire le tartre et l'accumulation de plaque sur les dents d'un animal de compagnie, méthode comprenant l'étape consistant à laisser un animal de compagnie mastiquer l'aliment déshydraté pour animaux de compagnie, l'aliment pour animaux de compagnie ayant une masse volumique inférieure à 330 Kgm⁻³, une longueur d'au moins 15 mm, une largeur d'au moins 13,5 mm et une épaisseur d'au moins 12 mm.

2. Aliment pour animaux de compagnie suivant la revendication 1, dans lequel la protéine dénaturée comprend, en poids de la composition alimentaire, 15 à 20 % de farine de gluten de maïs et 3 à 10 % de boeuf et de farine d'os.

3. Aliment pour animaux de compagnie suivant la revendication 1, comprenant environ 16 % en poids de farine de gluten de maïs, environ 14 % en poids de farine de soja, environ 4 % en poids de farines de poisson et de volaille, environ 8 % en poids de cellulose et de diverses vitamines et substances minérales, 62 % en poids de maïs entier et facultativement 2 % en poids de glycérol, ayant une masse volumique de 280 kg/m³.

4. Aliment déshydraté pour animaux de compagnie suivant les revendications 1 à 3, ledit aliment pour animaux de compagnie ayant une texture telle qu'une sonde ayant une surface de contact d'1 mm² et fonctionnant à une vitesse de 5 mm/s pénètre dans la matrice sur une distance d'au moins 30 % de l'épaisseur de la matrice avant rupture de la matrice.

5. Aliment pour animaux de compagnie suivant l'une quelconque des revendications précédentes, ledit aliment déshydraté pour animaux de compagnie ayant une activité d'eau inférieure à 0,7.

6. Aliment pour animaux de compagnie suivant l'une quelconque des revendications précédentes, ledit aliment déshydraté pour animaux de compagnie étant mélangé à un aliment humide pour animaux de compagnie.

7. Aliment pour animaux de compagnie suivant la revendication 6, dans lequel l'aliment déshydraté pour animaux de compagnie et l'aliment humide pour animaux de compagnie sont mélangés en un rapport de l'aliment déshydraté pour animaux de compagnie à l'aliment humide pour animaux de compagnie de 50:50.

8. Aliment pour animaux de compagnie suivant l'une quelconque des revendications 1 à 7, dans lequel la composition alimentaire pour animaux de compagnie est fournie sous des formes d'éléments broyés et pressés choisies entre des triangles, des éléments pentagonaux et des étoiles.

9. Composition alimentaire déshydratée pour animaux de compagnie comprenant 12 à 50 % de protéine dénaturée comprenant de la farine de gluten de blé, 2 à 15 % de fibre insoluble, 20 à 65 % d'un glucide gélatinisé et 0 à 5 % d'un agent humidifiant, l'aliment pour animaux de compagnie ayant une masse volumique inférieure à 330 kgm⁻³, et **caractérisée en ce que** l'aliment pour animaux de compagnie est fourni sous forme d'un élément broyé et pressé ayant une longueur d'au moins 15 mm, une largeur d'au moins 13,5 mm et une épaisseur d'au moins 12 mm.

10. Aliment déshydraté pour animaux de compagnie suivant la revendication 9, dans lequel la protéine dénaturée comprend, en poids de la composition alimentaire, 15 à 20 % de farine de gluten de maïs et 3 à 10 % de boeuf et de farine d'os.

11. Aliment déshydraté pour animaux de compagnie suivant la revendication 9 ou la revendication 10, dans lequel la protéine dénaturée comprend, en poids de la composition alimentaire, 5 à 15 % de farine de soja.

12. Composition alimentaire déshydratée pour animaux de compagnie suivant la revendication 9, comprenant environ 16 % en poids de farine de gluten de maïs, environ 14 % en poids de farine de soja, environ 4 % en poids de farines de poisson et de volaille, environ 3 % en poids de cellulose et de diverses vitamines et substances minérales, 62 % en poids de maïs entier et facultativement environ 2 % en poids de glycérol, ayant une masse volumique de 280 kg/m³.
